# EUROPEAN PATENT SPECIFICATION

(11) **EP 1 377 608 B1**
(45) Date of publication and mention of the grant of the patent: **16.09.2009**
(21) Application number: 02717999.3
(22) Date of filing: 21.03.2002
(51) Int. Cl.: C07K 14/62, C12P 21/02, C07K 19/00

(54) **INSULIN PRECURSORS AND A PROCESS FOR THEIR PREPARATION**
INSULINVORSTUFEN UND VERFAHREN ZU DEREN HERSTELLUNG
PRECURSEURS DE L'INSULINE ET PROCEDE DE PREPARATION

(30) Priority: 02.04.2001 DK 200100547
(43) Date of publication of application: 07.01.2004
(73) Proprietor: NOVO NORDISK A/S, 2880 Bagsvaerd (DK)
(72) Inventor: KJELDSEN, Thomas, Boerglum, DK-2830 Virum (DK)
(74) Representative: Secher, Anne
(86) International application number: PCT/DK2002/000190
(87) International publication number: WO 2002/079250

(56) References cited:
- EP-A2- 0 347 845
- WO-A1-01/49742
- WO-A1-01/49870
- US-A- 4 916 212
- US-A- 5 324 641
- US-A- 5 840 542
- US-A- 5 962 267

### SEQUENCE LISTING

<110> Novo Nordisk A/S
   Kjeldsen, Thomas
<120> Method For Making Human Insulin Precursors
<130> 6280.204-WO
<160> 12
<170> PatentIn version 3.1
<210> 1
   <211> 6
   <212> PRT
   <213> Artificial Sequence
<220>
   <223> N-terminal extension
   <400> 1
   Glu Glu Gly Glu Pro Lys
   1               5
<210> 2
   <211> 550
   <212> DNA
   <213> Artificial sequence
<220>
   <221> CDS
   <222> (115)..(486)
   <223>
<400> 2
   ttcttgctta aatctataac tacaaaaaac acatacagga attccattca agaatagttc      60
   aaacaagaag attacaaact atcaatttca tacacaatat aaacgattaa aaga atg     117
                                                               Met
                                                               1
   aaa ctg aaa act gta aga tct gcg gtc ctt tcg tca ctc ttt gca tct     165
   Lys Leu Lys Thr Val Arg Ser Ala Val Leu Ser Ser Leu Phe Ala Ser
               5                   10                  15
   cag gtc ctt ggc caa cca att gac gac act gaa tct caa act act tct     213
   Gln Val Leu Gly Gln Pro Ile Asp Asp Thr Glu Ser Gln Thr Thr Ser
           20                  25                  30
   gtc aac ttg atg gct gac gac act gaa tct gct ttc gct act caa act     261
   Val Asn Leu Met Ala Asp Asp Thr Glu Ser Ala Phe Ala Thr Gln Thr
       35                  40                  45
   aac tct ggt ggt ttg gat gtt gtt ggt ttg atc tcc atg gct aag aga     309
   Asn Ser Gly Gly Leu Asp Val Val Gly Leu Ile Ser Met Ala Lys Arg
   50                  55                  60                  65
   gaa gaa ggt gaa cca aag ttc gtt aac caa cac ttg tgc ggt tcc cac     357
   Glu Glu Gly Glu Pro Lys Phe Val Asn Gln His Leu Cys Gly Ser His
                   70                  75                  80
   ttg gtt gaa gct ttg tac ttg gtt tgc ggt gaa aga ggt ttc ttc tac     405
   Leu Val Glu Ala Leu Tyr Leu Val Cys Gly Glu Arg Gly Phe Phe Tyr
               85                  90                  95
   act cct aag gct gct aag ggt att gtc gaa caa tgc tgt acc tcc atc     453
   Thr Pro Lys Ala Ala Lys Gly Ile Val Glu Gln Cys Cys Thr Ser Ile
           100                 105                 110
   tgc tcc ttg tac caa ttg gaa aac tac tgc aac tagacgcagc ccgcaggctc   506
   Cys Ser Leu Tyr Gln Leu Glu Asn Tyr Cys Asn
       115                 120
   tagaaactaa gattaatata attatataaa aatattatct tctt                   550
<210> 3
   <211> 124
   <212> PRT
   <213> Artificial sequence
<400> 3
   Met Lys Leu Lys Thr Val Arg Ser Ala Val Leu Ser Ser Leu Phe Ala
   1               5                   10                  15
   Ser Gln Val Leu Gly Gln Pro Ile Asp Asp Thr Glu Ser Gln Thr Thr
               20                  25                  30
   Ser Val Asn Leu Met Ala Asp Asp Thr Glu Ser Ala Phe Ala Thr Gln
           35                  40                  45
   Thr Asn Ser Gly Gly Leu Asp Val Val Gly Leu Ile Ser Met Ala Lys
       50                  55                  60
   Arg Glu Glu Gly Glu Pro Lys Phe Val Asn Gln His Leu Cys Gly Ser
   65 70 75 80
   His Leu Val Glu Ala Leu Tyr Leu Val Cys Gly Glu Arg Gly Phe Phe
   85 90 95
   Tyr Thr Pro Lys Ala Ala Lys Gly Ile Val Glu Gln Cys Cys Thr Ser
   100 105 110
   Ile Cys Ser Leu Tyr Gln Leu Glu Asn Tyr Cys Asn
   115 120
<210> 4
   <211> 20
   <212> DNA
   <213> Artificial
<400> 4
   ttgcttaaat ctataactac 20
<210> 5
   <211> 105
   <212> DNA
   <213> Artificial
   <220>
   <221> misc_feature
   <222> (85)..(87)
   <223> n is A, C, T or G and m is A or C
<400> 5
   ttagtttcta gactagttgc agtagttttc caattggtac aaggagcaga tggaggtaca 60
   gcattgttcg acaataccct ttggmnnctt aggagtgtag aagaa 105
<210> 6
   <211> 44
   <212> PRT
   <213> Artificial
<400> 6
   Gln Pro Ile Asp Asp Thr Glu Ser Gln Thr Thr Ser Val Asn Leu Met
   1 5 10 15
   Ala Asp Asp Thr Glu Ser Ala Phe Ala Thr Gln Thr Asn Ser Gly Gly
   20 25 30
   Leu Asp Val Val Gly Leu Ile Ser Met Ala Lys Arg
   35 40
<210> 7
   <211> 45
   <212> PRT
   <213> Artificial
<400> 7
   Gln Pro Ile Asp Asp Thr Glu Ser Asn Thr Thr Ser Val Asn Leu Met
   1 5 10 15
   Ala Asp Asp Thr Glu Ser Arg Phe Ala Thr Asn Thr Thr Leu Ala Gly
   20 25 30
   Gly Leu Asp Val Val Asn Leu Ile Ser Met Ala Lys Arg
   35 40 45
<210> 8
   <211> 4
   <212> PRT
   <213> Artificial Sequence
<220>
   <223> C-peptide
   <400> 8
   Ser Asp Pro Lys
   1
<210> 9
   <211> 4
   <212> PRT
   <213> Artificial
<400> 9
   Asn Asp Pro Lys
   1
<210> 10
   <211> 4
   <212> PRT
   <213> Artificial Sequence
<220>
   <223> C-peptide
<400> 10
   Glu Asp Pro Lys
   1
<210> 11
   <211> 4
   <212> PRT
   <213> Artificial Sequence
<220>
   <223> C-peptide
   <400> 11
   Ala Asp Pro Lys
   1
<210> 12
   <211> 9
   <212> PRT
   <213> Artificial
<400> 12
   Glu Glu Ala Glu Ala Glu Ala Pro Lys
   1               5

## Description

### BACKGROUND

Yeast organisms produce a number of proteins that have a function outside the cell. Such proteins are referred to as secreted proteins. These secreted proteins are expressed initially inside the cell in a precursor or a pre-form containing a pre-peptide sequence ensuring effective direction (translocation) of the expressed product across the membrane of the endoplasmic reticulum (ER). The pre-peptide, normally named a signal peptide, is generally cleaved off from the desired product during translocation. Once entered in the secretory pathway, the protein is transported to the Golgi apparatus. From the Golgi, the protein can follow different routes that lead to compartments such as the cell vacuole or the cell membrane, or it can be routed out of the cell to be secreted to the external medium (Pfeffer at al. (1987) Ann. Rev. Biochem. 56:829-852).

Insulin is a polypeptide hormone secreted by β-cells of the pancreas and consists of two polypeptide chains, A and B, which are linked by two inter-chain disulphide bridges. Furthermore, the A-chain features one intra-chain disulphide bridge.

The hormone is synthesized as a single-chain precursor proinsulin (preproinsulin) consisting of a prepeptide of 24 amino acid followed by proinsulin containing 86 amino acids in the configuration: prepeptide - B - Arg Arg - C - Lys Arg -A, in which C is a connecting peptide of 31 amino acids. Arg-Arg and Lys-Arg are cleavage sites for cleavage of the connecting peptide from the A and B chains.

Three major methods have been used for the production of human insulin in microorganisms. Two involve *Escherichia coli*, with either the expression of a large fusion protein in the cytoplasm (Frank et al. (1981) in Peptides: Proceedings of the 7th American Peptide Chemistry Symposium (Rich & Gross, eds.), Pierce Chemical Co., Rockford, IL. pp 729-739), or use of a signal peptide to enable secretion into the periplasmic space (Chan et al. (1981) PNAS 78:5401-5404). A third method utilizes *Saccharomyces cerevisiae* to secrete an insulin precursor into the medium (Thim et al. (1986) PNAS 83:6766-6770). The prior art discloses a limited number of insulin precursors which are expressed in either *E. coli* or *Saccharomyces cerevisiae*, vide US 5,962,267, WO 95/16708, EP 0055945, EP 0163529, EP 0347845 and EP 0741188.

### SUMMARY OF THE INTENTION

The present invention features novel connecting peptides (C-peptides) which confer an increased production yield of insulin precursor molecules when expressed in a transformed microorganism, in particular yeast. Such insulin precursors can then be converted into human insulin, desB30 human insulin or certain acylated human insulins by one or more suitable, well known conversion steps.

The connecting peptides of the present invention contain at least one Pro and will generally be relatively short, typically not longer than 6 - 4 amino acid residues in length.

The connecting peptide contains a cleavage site at its C-terminal end enabling in vitro cleavage of the connecting peptide from the A chain. The cleavage site enabling cleavage of the connecting peptide from the A-chain is a single basic amino acid residue Lys or Arg, preferably Lys which is cleavable by trypsin or trypsin like proteases; *Acromobactor lyticus* protease or by a carboxypeptidase protease.

Cleavage of the connecting peptide from the B chain is enabled by cleavage at the natural Lys^{B29} amino acid residue in the B chain giving rise to a desB30 insulin precursor. If the insulin precursor is to be converted into human insulin, the B30 Thr amino acid residue (Thr) can then be added by well known in vitro, enzymatic procedures. The desB30 insulin may also be converted into an acylated insulin as disclosed in US 5,750,497 and US 5,905,140.

In one embodiment the insulin precursors according to the invention comprise the formula B(1-29) - X₁ - Y - A(1-21), wherein X₁ is a peptide sequence of 1 - 5 amino acid residues of which at least one is Pro, and Y is Lys or Arg.

In another embodiment of the present invention the Pro is immediately N-terminal to the cleavage site Y adjacent to the A chain. The connecting peptide may contain more than one Pro, but preferably not more than three Pro residues.

In one embodiment, the total number of amino acid residues in X₁ will be from 1 - 4, 1-3, or 1-2 amino acid residues. The amino acid residues in X₁ can be any codable amino acid residue and may be the same or different with the only proviso that at least one is Pro.

In a further embodiment the sequence X₁ - Y will not contain two adjacent basic amino acid residues (Lys, Arg) and in a still further embodiment X₁ will contain an amino acid residue with a free COOH group (Glu, Asp) or an amino acid residue with a free amino group (Gln, Asn, His).

Examples of X₁ - Y - sequences are GlnProLys; ThrProLys; GluProLys; GlyProLys; MetProLys; SerProLys; AspProLys; AlaAspProLys (SEQ ID NO:8); AsnAspProLys (SEQ ID NO:9); GluAspProLys (SEQ ID NO:10) and AlaAspProLys (SEQ ID NO:11).

The present invention is also related to polynucleotide sequences which code for the claimed insulin precursors.

In another aspect, the invention relates to a process for making an insulin precursor said method comprising
(i) culturing a yeast cell comprising a polynucleotide sequence encoding an insulin precursor with the formula B(1-29) - X₁ - Y - A(1-21) wherein X₁ is a peptide sequence of 1 - 5 amino acid residues of which at least one is Pro and Y is Lys or Arg, under suitable culture conditions for expression of said insulin precursor; and
(ii) isolating the expressed insulin precursor.

In still a further aspect, the invention relates to a process for making desB30 human insulin or human insulin, said method comprising
(i) culturing a yeast cell comprising a polynucleotide sequence encoding an insulin precursor with the formula B(1-29) - X₁ - Y - A(1-21) wherein X₁ is a peptide sequence of 1 - 5 amino acid residues of which at least one is Pro and Y is Lys or Arg, under suitable culture conditions for expression of said insulin precursor;
(ii) isolating the insulin precursor from the culture medium and
(iii) converting the insulin precursor into desB30 human insulin or human insulin by in vitro chemical or enzymatic conversion.

In one embodiment of the present invention the host cell is a yeast host cell and in a further embodiment the yeast host cell is selected from the genus *Saccharomyces*. In a further embodiment the yeast host cell is selected from the species *Saccharomyces cerevisiae*.

### BRIEF DESCRIPTION OF THE DRAWINGS

Fig. 1 represents the pAK855 *S. cerevisiae* expression plasmid expressing the TA57 leader-EEGEPK(SEQ ID NO:1-)-B(1-29)-AlaAlaLys-A(1-21) precursor
Fig. 2 represents the nucleotide sequence of the expression cassette of the pAK855 yeast expression plasmid and the inferred amino acid sequence (SEQ ID NO: 2 and 3).

### DETAILED DESCRIPTION

### Abbreviations and nomenclature.

By "**connecting peptide**" or "**C-peptide**" is meant the connection moiety "**C**" of the B-C-A polypeptide sequence of a single chain preproinsulin-like molecule. Specifically, in the natural insulin chain, the C-peptide connects position 30 of the B chain and position 1 of the A chain. A "mini C-peptide" or "connecting peptide" such as those described herein, connect B29 to A1 and differ in sequence and length from that of the natural C-peptide.

By "**IP**" is meant a singte-chain insulin precursor in which a desB30 chain is linked to the A chain of insulin via a connecting peptide. The single-chain insulin precursor will contain correctly positioned disulphide bridges (three) as in human insulin.

With "desB30" or "B(1-29)" is meant a natural insulin B chain lacking the B30 amino acid residue and "A(1-21)" means the natural insulin A chain. The mini C-peptide and its amino acid sequence are indicated in the three letter amino acid code.

By "**insulin precursor**" is meant a single-chain polypeptide which by one or more subsequent chemical and/or enzymatic processes can be converted into human insulin or desB30 human insulin.

The term "**immediately N-terminal to**" is meant to illustrate the situation where an amino acid residue or a peptide sequence is directly linked at its C-terminal end to the N-terminal end of another amino acid residue or amino acid sequence by means of a peptide bond.

The present invention features novel mini C-peptides connecting position 29 of the insulin B chain and position 1 of the insulin A chain which significantly increased production yields in a yeast host cell. By the term "**significantly increased production,**" **"increased fermentation yield,**" and the like, is meant an increase in secreted amount of the insulin precursor molecule present in the culture supernatant compared to the yield of an insulin precursor with a C peptide different from the C peptides according to the present invention. An **"increased"** fermentation yield is an absolute number larger than the control; preferably, the increase is 50% or more larger than the control; even more preferably, the increase is 100% or more larger than control levels.

"***POT***" is the *Schizosaccharomyces pombe* triose phosphate isomerase gene, and "***TPI1***" is the *S. cerevisiae* triose phosphate isomerase gene.

By a "leader" is meant an amino acid sequence consisting of a pre-peptide (the signal peptide) and a pro-peptide.

The term **"signal peptide"** is understood to mean a pre-peptide which is present as an N-terminal sequence on the precursor form of a protein. The function of the signal peptide is to allow the heterologous protein to facilitate translocation into the endoplasmic reticulum. The signal peptide is normally cleaved off in the course of this process. The signal peptide may be heterologous or homologous to the yeast organism producing the protein. A number of signal peptides which may be used with the DNA construct of the invention including yeast aspartic protease 3 (YAP3) signal peptide or any functional analog (Egel-Mitani et al. (1990) YEAST 6.127-137 and US 5,726,038) and the α-factor signal of the *MF*α*1* gene (Thorner (1981) in The Molecular Biology of the Yeast Saccharomyces cerevisiae, Strathem et al., eds., pp 143-180, Cold Spring Harbor-Laboratory, NY and US 4,870,00.

The term "**pro-peptide**" means a polypeptide sequence whose function is to allow the expressed polypeptide to be directed from the endoplasmic reticulum to the Golgi apparatus and further to a secretory vesicle for secretion into the culture medium (i.e. exportation of the polypeptide across the cell wall or at least through the cellular membrane into the periplasmic space of the yeast cell). The pro-peptide may be the yeast α-factor pro-peptide, vide US 4,546,082 and 4,870,008. Alternatively, the pro-peptide may be a synthetic pro-peptide, which is to say a pro-peptide not found in nature. Suitable synthetic pro-peptides are those disclosed in US 5,395,922; 5,795,746; 5,162,498 and WO 98/32867. The pro- peptide will preferably contain an endopeptidase processing site at the C-terminal end, such as a Lys-Arg sequence or any functional analog thereof.

The polynucleotide sequence of the invention may be prepared synthetically by established standard methods, e.g. the phosphoamidite method described by Beaucage et al. (1981) Tetrahedron Letters 22:1859-1869, or the method described by Matthes et al. (1984) EMBO Journal 3:801-805. According to the phosphoamidite method, oligonucleotides are synthesized, for example, in an automatic DNA synthesizer, purified, duplexed and ligated to form the synthetic DNA construct. A currently preferred way of preparing the DNA construct is by polymerase chain reaction (PCR).

The polynucleotide sequence of the invention may also be of mixed genomic, cDNA, and synthetic origin. For example, a genomic or cDNA sequence encoding a leader peptide may be joined to a genomic or cDNA sequence encoding the A and B chains, after which the DNA sequence may be modified at a site by inserting synthetic oligonucleotides encoding the desired amino acid sequence for homologous recombination in accordance with well-known procedure or preferably generating the desired sequence by PCR using suitable oligonucleotides.

The invention encompasses a vector which is capable of replicating in the selected microorganism or host cell and which carries a polynucleotide sequence encoding the insulin precursors of the invention. The recombinant vector may be an autonomously replicating vector, *i*.*e*., a vector which exists as an extra-chromosomal entity, the replication of which is independent of chromosomal replication, *e*.*g*., a plasmid, an extra-chromosomal element, a mini-chromosome, or an artificial chromosome. The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used. The vector may be linear or closed circular plasmids and will preferably contain an element(s) that permits stable integration of the vector into the host cell's genome or autonomous replication of the vector in the cell independent of the genome.

In a preferred embodiment, the recombinant expression vector is capable of replicating in yeast. Examples of sequences which enable the vector to replicate in yeast are the yeast plasmid'2 µm replication genes REP 1-3 and origin of replication.

The vectors of the present invention preferably contain one or more selectable markers which permit easy selection of transformed cells. A selectable marker is a gene the product of which provides for biocide or viral resistance, resistance to heavy metals, prototrophy to auxotrophs, and the like. Examples of bacterial selectable markers are the *dal* genes from *Bacillus subtilis* or *Bacillus licheniformis*, or markers which confer antibiotic resistance such as ampicillin, kanamycin, chloramphenicol or tetracycline resistance. Selectable markers for use in a filamentous fungal host cell inlude *amdS* (acetamidase), *argB* (ornithine carbamoyltransferase), *pyrG* (orotidine-5'-phosphate decarboxylase) and *trpC* (anthranilate synthase. Suitable markers for yeast host cells are ADE2, HIS3, LEU2, LYS2, MET3, TRP1, and URA3. A preferred selectable marker for yeast is the *Schizosaccharomyces pompe* TPI gene (Russell (1985) Gene 40:125-130).

In the vector, the polynucleotide sequence is operably connected to a suitable promoter sequence. The promoter may be any nucleic acid sequence which shows transcriptional activity in the host cell of choice including mutant, truncated, and hybrid promoters, and may be obtained from genes encoding extra-cellular or intra-cellular polypeptides either homologous or heterologous to the host cell.

Examples of suitable promoters for directing the transcription in a bacterial host cell, are the promoters obtained from the *E. coli. lac* operon, *Streptomyces coelicolor* agarase gene (*dagA*), *Bacillus subtilis* levansucrase gene (*sacB*), *Bacillus licheniformis* alpha-amylase gene (*amyL*), *Bacillus stearothermophilus* maltogenic amylase gene (*amyM*) *Bacillus amyloliquefaciens* alpha-amylase gene (*amyQ*), and *Bacillus licheniformis* penicillinase gene (*penP*). Examples of suitable promoters for directing the transcription in a filamentous fungal host cell are promoters obtained from the genes for *Aspergillus oryzae* TAKA amylase, *Rhizomucor miehei* aspartic proteinase, *Aspergillus niger* neutral alpha-amylase, and *Aspergillus niger* acid stable alpha-amylase. In a yeast host, useful promoters are the *Saccharomyces cerevisiae* Ma1, TPI, ADH or PGK promoters.

The polynucleotide construct of the invention wilt also typically be operably connected to a suitable terminator. In yeast a suitable terminator is the TPI terminator (Alber et al. (1982) J. Mol. Appl. Genet. 1:419434).

The procedures used to ligate the polynucleotide sequence of the invention, the promoter and the terminator, respectively, and to insert them into a suitable vector containing the information necessary for replication in the selected host, are well known to persons skilled in the art. It will be understood that the vector may be constructed either by first preparing a DNA construct containing the entire DNA sequence encoding the insulin precursors of the invention, and subsequently inserting this fragment into a suitable expression vector, or by sequentially inserting DNA fragments containing genetic information for the individual elements (such as the signal, pro-peptide, mini C-peptide, A and B chains) followed by ligation.

The present invention also relates to recombinant host cells, comprising a polynucleotide sequence encoding the insulin precursors of the invention. A vector comprising such polynucleotide sequence is introduced into the host cell so that the vector is maintained as a chromosomal integrant or as a self-replicating extra-chromosomal vector as described earlier. The term "host cell" encompasses any progeny of a parent cell that is not identical to the parent cell due to mutations that occur during replication. The host cell may be a unicellular microorganism, *e*.*g*., a prokaryote, or a non-unicellular microorganism, *e*.*g*., a eukaryote. Useful unicellular cells are bacterial cells such as gram positive bacteria including, but not limited to, a *Bacillus* cell, *Streptomyces* cell, or gram negative bacteria such as *E. coli* and *Pseudomonas* sp. Eukaryote cells may be mammalian, insect, plant, or fungal cells. In a preferred embodiment, the host cell is a yeast cell. The yeast organism used in the process of the invention may be any suitable yeast organism which, on cultivation, produces large amounts of the insulin precursor and insulin precursor analogs of the invention. Examples of suitable yeast organisms are strains selected from the yeast species *Saccharomyces cerevisiae, Saccharomyces kluyveri, Schizosaccharomyces pombe, Sacchoromyces uvarum, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp*., and *Geotrichum fermentans*.

The transformation of the yeast cells may for instance be effected by protoplast formation followed by transformation in at manner known per se. The medium used to cultivate the cells may be any conventional medium suitable for growing yeast organisms. The secreted insulin precursor of the invention, a significant proportion of which will be present in the medium in correctly processed form, may be recovered from the medium by conventional procedures including separating the yeas cells from the medium by centrifugation, filtration or catching the insulin precursor by an ion exchange matrix or by a reverse phase absorption matrix, precipitating the proteinaceous components of the supernatant or filtrate by means of a salt, e.g. ammonium sulphate, followed by purification by a variety of chromatographic procedures, e.g. ion exchange chromatography, affinity chromatography, or the like.

The insulin precursors of the invention may be expressed with an N-terminal amino acid residue extension, as described in U.S. Patent No. 5,395,922 and European Patent No. 765,395A, both of which patents are herein specifically incorporated by reference. The extension is found to be stably attached to the insulin precursor of the invention during fermentation, protecting the N-terminal end of the insulin precursor or insulin precursor analogue against the proteolytic activity of yeast proteases such as DPAP. The presence of an N-terminal extension on the insulin precursor may also serve as a protection of the N-terminal amino group during chemical processing of the protein, i.e. it may serve as a substitute for a BOC (t-butyl-oxycarbonyl) or similar protecting group. The N-terminal extension may be removed from the recovered insulin precursor by means of a proteolytic enzyme which is specific for a basic amino acid (e.g., Lys) so that the terminal extension is cleaved off at the Lys residue. Examples of such proteolytic enzymes are trypsin or *Achromobacter lyticus* protease.

After secretion to the culture medium and recovery, the insulin precursor of the invention will be subjected to various in vitro procedures to remove the possible N-terminal extension sequence and the mini C-peptide to give desB30 insulin. DesB30 insulin may then be converted into human insulin by adding a Thr in position B30. Conversion of the insulin precursor into human insulin by a suitable enzymatic conversion by means of trypsin or an *Achromobacter lyticus* protease in the presence of an L-threonine ester followed by conversion of the threonine ester of the insulin into insulin by basic or acid hydrolysis as described in US patent specification No. 4,343,898 or 4,916,212 or Research Disclosure, September 1994/487 the disclosures of which are incorporated by reference hereinto. DesB30 insulin may also be converted into an acylated derivative as disclosed in US 5,750,497 and US 5,905,140 the disclosures of which are incorporated by reference hereinto.

As described below, insulin precursors with synthetic C-peptides were constructed featuring at least one Pro. *Saccharomyces cerevisiae* expression plasmids containing a polynucleotide sequence encoding the claimed insulin precursors were constructed by PCR and used to transform a *S. cerevisiae* host cell. The amount of expressed product was measured as a percentage of the amount of expressed control. The novel C-peptides of the invention gave increased yields of up to 100%.

The present process allows production of an acylated desB30 human insulin by culturing a host cell comprising a polynucleotide sequence encoding an insulin precursor of the invention; isolating the insulin precursor from the culture medium, converting the insulin precursor into desB30. human insulin and converting the desB30 human insulin into an acylated derivate by use of a convenient acylation method.

The present invention is described in further detain in the following examples which are not in any way intended to limit the scope of the invention as claimed. The attached Figures are meant to be considered as integral parts of the specification and description of the invention.

### EXAMPLES

### General Procedures

All expressions plasmids are of the C-POT type, similar to those described in EP 171, 142, which are characterized by containing the *Schizosaccharomyces pombe* triose phosphate isomerase gene (POT) for the purpose of plasmid selection and stabilization in *S. cerevisiae.* The plasmids also contain the *S. cerevisiae* triose phosphate isomerase promoter and terminator: These sequences are simitar to the corresponding sequences in plasmid pKFN1003 (described in WO 90/100075) as are all sequences except the sequence of the *Eco*RI-*Xba*I fragment encoding the fusion protein of the leader and the insulin precursor product. In order to express different fusion proteins, the *Eco*RI-*Xba*I fragment of pKFN1003 is simply replaced by an *Eco*RI-*Xba*I fragment encoding the leader-insulin precursor-fusion of interest. Such *Eco*RI-*Xba*I fragments may be synthesized using synthetic oligonucleotides and PCR according to standard techniques.

Yeast transformants were prepared by transformation of the host strain *S. cerevisiae* strain M663 (*MATalMAT*α *pep4-*3/*pep4*-3 *HIS4*/*his4 tpi::LEU2*/*tpi::LEU2* Cir⁺). The yeast strain MT663 was deposited in the Deutsche Sammlung von Mikroorganismen und Zellkulturen in connection with filing WO 92/11378 and was given the deposit number DSM 6278.

MT663 was grown on YPGaL (1% Bacto yeast extract, 2% Bacto peptone, 2% galactose, 1% lactate) to an O.D. at 600 nm of 0.6 100 ml of culture was harvested by centrifugation, washed with 10 ml of water, recentrifuged and resuspended in 10 ml of a solution containing 1.2 M sorbitol, 25 mM Na₂EDTA pH = 8.0 and 6.7 mg/ml dithiotreitol. The suspension was incubated at 30°C for 15 minutes, centrifuged and the cells resuspended in 10 ml of a solution containing 1.2 M sorbitol, 10 mM Na₂EDTA, 0.1 M sodium citrate, pH 0 5.8, and 2 mg Novozym®234. The suspension was incubated at 30°C for 30 minutes, the cells collected by centrifugation, washed in 10 ml of 1.2 M sorbitol and 10 ml of CAS (1.2 M sorbitol, 10 mM CaCl₂, 10 mM Tris HCl (Tis = Tris(hydroxymethyl)aminomethane) pH = 7.5) and resuspended in 2 ml of CAS. For transformation, 1 ml of CAS-suspended cells was mixed with approx. 0.1 mg of plasmid DNA and left at room temperature for 15 minutes. 1 ml of (20% polyethylene glycol 4000, 10 mM, CaCl₂, 10 mM Tris HCl, pH = 7.5) was added and the mixture left for a further 30 minutes at room temperature. The mixture was centrifuged and the pellet resuspended in 0.1 ml of SOS (1.2 M sorbitol, 33% v/v YPD, 6.7 mM CaCl₂) and incubated at 30°C for 2 hours. The suspension was then centrifuged and the pellet resuspended in 0.5 ml of 1.2, M, sorbitol. Then, 6 ml of top agar (the SC medium of Sherman et al. (1982) Methods in Yeast Genetics, Cold Spring Harbor Laboratory) containing 1.2 M sorbitol plus 2.5% agar) at 52°C was added and the suspension poured on top of plates containing the same agar-solidified, sorbitol containing medium.

*S. cerevisiae* strain MT663 transformed with expression plasmids was grown in YPD for 72 h at 30°C. Quantitation of the insulin-precursor yield in the culture supernatants was performed by reverse-phase HPLC analysis with human insulin as an external standard (Snel &.Damgaard (1988) Proinsulin heterogenity in pigs. Horm. Metabol. Res. 20:476-488).

### Example 1

Synthetic genes encoding fusion proteins, consisting of the insulin precursor associated with a leader sequence consisting of a pre-peptide (signal peptide) and a pro-peptide, were constructed using PCR under standard conditions (Sambrook et al. (1989) Molecular Cloning, Cold Spring Harbor Laboratory Press) and E.H.F. polymerase (Boehringer Mannheim GmbH, Sandhoefer Strasse 116, Mannheim, Germany). The resulting DNA fragments were isolated and digested with endonucleases and purified using the Gene Clean kit (Bio101 Inc., La Jolla, CA, USA). Standard methods were used for DNA ligation and transformation of *E*. *coli* cells were performed by the CaCl₂ method (Sambrook et al. (1989) *supra*)*.* Plasmids were purified from transformed *E. coli* cells using QIAGEN columns (QIAGEN, Hilden, Germany). Nucleotide sequences were determined using the ALF Pharmacia Biotech DNA sequencing system with purified double-stranded plasmid DNA as template. Oligonucleotide primers for PCR were obtained from DNA technology (Arhus, Denmark):

Secretion of the insulin precursor was facilitated by the TA57 leader or the TA39 leader (Kjeldsen et al., 1998. Protein Expression Purif. 14, 309-316), although a variety of known yeast leader sequences may be used.

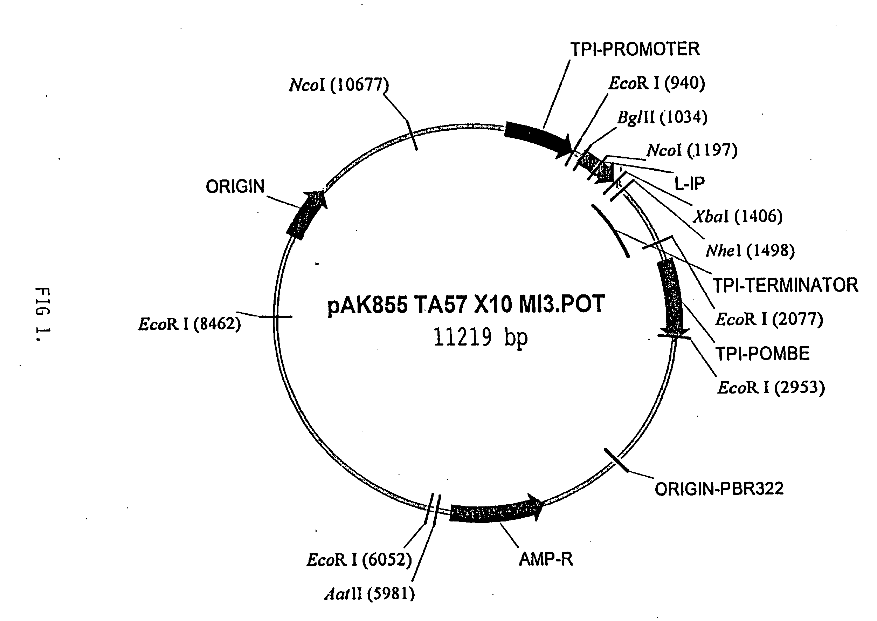

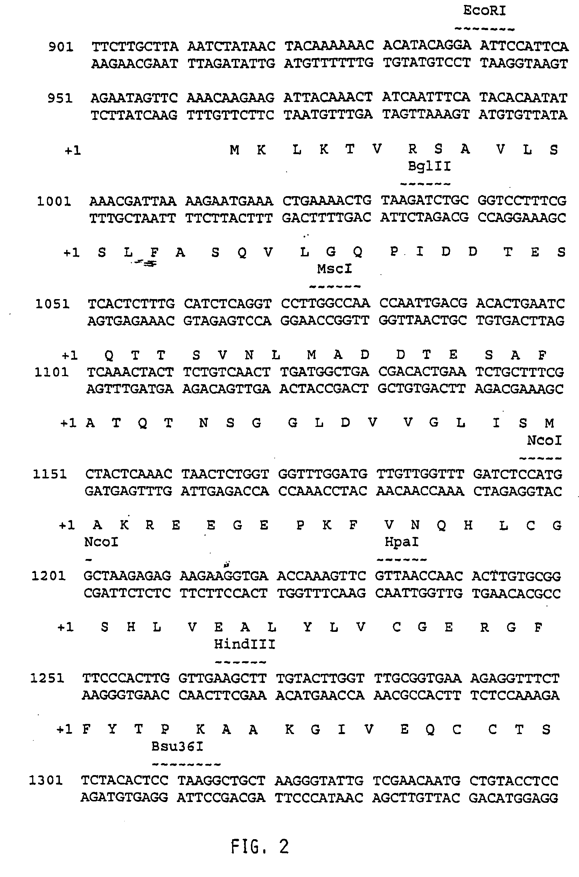

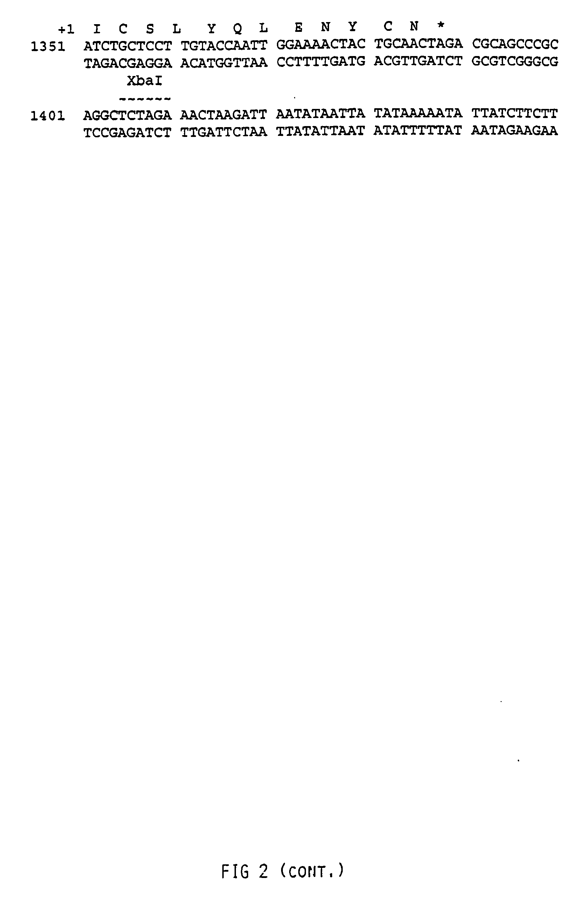

As shown in Fig. 1 and 2, the pAK855 *S. cerevisiae* expression plasmid expressing the TA57 leader-EEGEDK(SEQ ID NO:1)-insulin precursor fusion protein was constructed based on the *S. cerevisiae-E. coli* shuttle *POT* plasmid (U.S. patent 5,871,957). In Fig. 1 L-IP indicates the fusion protein expression cassette encoding the leader-insulin precursor fusion protein; TPI-PROMOTER is the *S*. *cerevisiae TPI1*, promoter, TPI-TERMINATOR is the *S*. *cerevisiae TPI1* terminator; TPI-POMBE indicates the *S. pombe POT* gene used for selection in *S. cerevisiae*; ORIGIN indicates a *S. cerevisiae* origin of replication derived from the 2µm plasmid; AMP-R indicates the β-lactamase gene conferring resistance toward ampicillin, facilitating selection in *E. coli*; and ORIGIN-PBR322 indicates an *E. coli* origin of replication.

DNA encoding a number of fusions proteins of leader sequences and insulin precursors with different mini C-peptides was generated by PCR using appropriate oligonucleotides as primers, as described below. Standard methods were used to subclone DNA fragments encoding the leader-insulin precursor-fusion proteins into the CPOT expression vector in the following configuration: leader-Lys-Arg-spacer-insulin precursor, where Lys-Arg is a potential dibasic endoprotease processing site and spacer is an N-terminal extension. To optimize processing of the fusion protein by the *S. cerevisiae* Kex2 endopcotease, DNA encoding a spacer peptide (N-terminal extension), e.g. EEGEPK (SEQ ID NO:1), was inserted between the DNA encoding the leader and the insulin precursor (Kjeldsen, et al.1999b. J. Biotechnology, 75, 195-208). However, the present of the spacer peptide is not mandatory. The insulin precursor was secreted as a single-chain N-terminally extended insulin precursor with a mini C-peptide, connecting Lys^{B29} and Gly^{A1}. After purification of the insulin precursor and proteolytic removal of the N-terminal extension and the mini C-peptide, the amino acid Thr^{B30} can be added to Lys^{B29} by enzyme-mediated transpeptidation, to generate human insulin (Markussen, et al. (1987) in "Peptides 1986" (Theodoropoulos, D:, Ed.), pp. 189-194, Walter de Gruyter & Co., Berlin.).

Development of synthetic mini C-peptides was performed by randomization of one or more codon(s) encoding the amino acids in the mini C-peptide. The synthetic mini C-peptides feature typically an enzymatic processing site (Lys) at the C-terminus which allows enzymatic removal of the synthetic mini C-peptide. Randomization was performed using doped oligonucleotides which introduced codon(s) variations at one or more positions of the synthetic mini C-peptides. Typically one of the two primers (oligonucleotides) used for PCR was doped. An example of an oligonucleotides pair used for PCR generation of leader-insulin precursor with randomized synthetic mini C-peptides used to generated synthetic mini C-peptides with the general formula: Xaa-Pro-Lys (XPK) are as follows:
Primer A:
   5'-TTGCTTAAATCTATAACTAC-3' (SEQ ID NO:4)
Primer B:
N=ACTG
M=AC

*Polymerase chain reaction.* PCR was typically performed as indicated below: 5 µl Primer A (20 pmol), 5 µl Primer B (20 pmol), 10 µl 10X PCR buffer, 8 µl dNTP mix, 0.75 µl E.H.F. enzyme, 1 µl pAK855 plasmid as template (approximately 0.2, µg DNA) and 70.25 µl distilled water.

Typically between 10 and 15 cycles were performed, one cycle typically was 95° C for 45 sec.; 55° C for 1 min; 72°. C for 1.5 min. The PCR mixture was subsequently loaded onto a 2 % agarose gel and electrophoresis was performed using standard techniques, The resulting DNA fragment was cut out of the agarose gel and isolated by the Gene Clean kit.

Fig. 2 shows the nucleotide sequence of pAK855 DNA expression cassette used as template for PCR and inferred amino acids of the encoded fusion protein (TA57-leader-EEGEPK(SEQ ID NO:1)-insulin precursor of pAK855 (SEQ ID NO: 2 and 3).

Likewise expression cassettes encoding a fusion protein TA39-leader-EEGEEPK(SEQ ID NO:1)-insulin precursor

The purified PCR DNA fragment was dissolved in water and restriction endonucleases buffer and digested with suitable restriction endonucleases (e.g. Bgl II and Xba I) according to standard techniques. The BgIII-XbaI DNA fragments were subjected to agarose electrophoresis and purified using The Gene Clean Kit.

The digested and isolated DNA fragments were ligated together with a suitable vector (e.g. of the CPOT type) using T4 DNA ligase and standard conditions. The ligation mix was subsequently transformed into a competent E. coli strain followed by selection with ampicillin resistance. Plasmids from the resulting *E. coli's* were isolated using QIAGEN columns.

The plasmids were subsequently used for transformation of a suitable *S. cerevisiae* host strain, e.g., MT663 (*MATalMAT*α *pep4*-3/*pep4*-3*. HIS4*/*his4 tpi::LEU2*/*tpi::LEU2* Cir⁺). Individual transformed *S. cerevisiae* clones were grown in liquid culture, and the quantity of the insulin precursor secreted to the culture supernatants was determined by RP-HPLC. The DNA sequence encoding the synthetic mini C-peptide of the expression plasmids from *S. cerevisiae* clones secreting increased quantity of the insulin precursor were then determined. Subsequently, the identified synthetic mini C-peptide sequence might be subjected to another round of randomization optimization.

Table 1 shows the insulin precursors generated by the above method and production yield expressed as a percent of control. Fermentation was conducted at 30°C for 72 h in 5 ml YPD. Yield of the insulin precursor was determined by RP-HPLC of the culture supernatant, and is expressed relative to the yield of a control strain expressing a leader-insulin precursor fusion protein in which the B29 residue is linked to the A1 residue by a mini C-peptide Ala-Ala-Lys. YAP3 is the YAP3 signal sequence. The sequence EEGEPK (SEQ ID NO:1) is an N-terminal extension to the B-chain and TA57 is a synthetic pro-sequence QPIDDTESQTTSVNLMADDTESAFATQTNSGGLDWGLISMAKR (SEQ ID NO:6).

**Table 1**

| Leader-N-terminal extension | Precursor | mini C-peptide | Yield* |
|---|---|---|---|
| YAP3-TA57-EEGEPK (SEQ ID NO:1) | IP (control) | AlaAlaLys | 100 |
| YAP3-TA57-EEGEPK (SEQ ID NO:1) | IP | GlnproLys, | 190 |
| YAP3-TA57-EEGEPK (SEQ ID NO:1) | IP | ThrProLys | 125 |
| YAP3-TA57-EEGEPK (SEQ ID NO:1) | IP | GluProLys | 164 |
| YAP3-TA57-EEGEPK (SEQ IO NO:1) | IP | GlyProLys | 120 |
| YAP3-TA57-EEGEPK (SEQ ID NO:1) | IP | MetProLys | 118 |
| YAP3-TA57-EEGEPK (SEQ ID NO:1) | IP | SerProLys | 107 |
| YAP3-TA57-EEGEPK (SEQ ID NO:1.) | IP | AspProLys | 187 |

### Example 2

By procedures analogue to the procedures described in Example 1, insulin precursors with a Pro in the mini C-peptide under the control of the synthetic leader TA37 QPIDDTESNTTSVNLMADDTESRFATNTTLAGGLDVVNLI-SMAKR (SEQ ID NO:7) and the YAP3 signal sequence were expressed in the yeast host MT663. The precursors all comprised an N-terminal extension EEGEPK (SEQ. ID NO:1). The increase in expression yield compared to a control is shown in Table 2.

**Table 2**

| Leader-N-terminal extension | Precursor | mini C-peptide | Yield* |
|---|---|---|---|
| YAP3-TA39-EEGEPK (SEQ ID NO:1) | IP (control) | AlaAlaLys | 100 |
| YAP3-TA39-EEGEPK (SEQ ID NO;1) | IP | SerAspProLys (SEQ ID NO:8) | 124 |
| YAP3-TA39-EEGEPK (SEQ ID NO:1) | IP | AsnAspProLys (SEQ ID NO:9) | 135 |
| YAP3-TA39-EEGEPK (SEQ ID NO:1) | IP | GluAspProLys (SEQ ID NO:10) | 130 |
| YAP3-TA39-EEGEPK (SEQ ID NO:1) | IP | AlaAspProLys (SEQ ID NO:11) | 130 |

### Example 3

By procedures analogue to the procedures described in Example 1, insulin precursors with a Pro in the mini C-peptide under the control of the α-factor leader sequence were expressed in the yeast host MT663. The insulin precursors were expressed with and without an N-terminal extension EEAEAEAPK (SEQ ID NO:12). The increase in expression yield compared to a control is shown in Table 3.

**Table 3**

| Leader-N-terminal extension | Precursor | mini C-peptide | Yield* |
|---|---|---|---|
| α-factor-EEAEAEAPK (SEQ ID NO:12) | IP (control) | AlaAlaLys | 100 |
| α-factor-EEAEAEAPK (SEQ ID NO:12) | IP | AspProLys | 211 |
| α-factor | IP | AspProLys | 289 |

## Claims

1. An insulin precursor comprising the formula
B(1-29) - X₁ - Y - A(1-21)
wherein
X₁ is a peptide sequence of 1 - 5 amino acid residues of which at least one is Pro and
Y is Lys or Arg.

2. An insulin precursor according to claim 1, wherein X₁ is 1-4 amino acid residues.

3. An insulin precursor according to claim 1, wherein X₁ is 1-3 or 1-2 amino acid residues.

4. An insulin precursor according to claim 1, wherein X₁ is GluPro and Y is Lys.

5. An insulin precursor according to claim 1, wherein X₁ is AspPro and Y is Lys.

6. An insulin precursor according to claim 1, wherein X₁ is GInPro and Y is Lys.

7. An insulin precursor according to one of claims 1 - 6, wherein one Pro is immediately N-terminal to Y.

8. A polynucleotide sequence encoding an insulin precursor with the formula
B(1-29) - X₁ - Y - A(1-21)
wherein
X₁ is a peptide sequence of 1 - 5 amino acid residues of which at least one is Pro and
Y is a Lys or Arg.

9. A process for making an insulin precursor said method comprising
(i) culturing a yeast cell comprising a polynucleotide sequence encoding an insulin precursor with the formula
B(1-29) - X₁ - Y - A(1-21)
wherein
X₁ is a peptide sequence of 1 - 5 amino acid residues of which at least one is Pro and
Y is Lys or Arg,
under suitable culture conditions for expression of said insulin precursor; and
(ii) isolating the expressed insulin precursor.

10. A process for making desB30 human insulin or human insulin, said method comprising
(i) culturing a yeast cell comprising a polynucleotide sequence encoding an insulin precursor with the formula
B(1-29) - X₁ - Y - A(1-21)
wherein
X₁ is a peptide sequence of 1 - 5 amino acid residues of which at least one is Pro and
Y is Lys or Arg,
under suitable culture conditions for expression of said insulin precursor;
(ii) isolating the insulin precursor from the culture medium and
(iii) converting the insulin precursor into desB30 human insulin or human insulin by in vitro chemical or enzymatic conversion.

11. A process according to claim 10, wherein X₁ is 1-4 amino acid residues.

12. A process according to claim 10, wherein X₁ is 1-3 amino acid residues.

13. A process according to claim 10, wherein X₁ is 1-2 amino acid residues.

14. A process according to one of claims 10-13, wherein one Pro is immediately N-terminal to Y.

15. A process according to claim 10, wherein X₁ is GluPro and Y is Lys.

16. A process according to claim 10 wherein X₁ is AspPro and Y is Lys.

17. A process according to claim 10, wherein X₁ is GlnPro and Y is Lys.

## Patentansprüche

1. Insulin-Vorläufer, umfassend die Formel
B(1-29) - X₁ - Y - A(1-21)
wobei
X₁ für eine Peptidsequenz von 1-5 Aminosäureresten, unter welchen es sich bei mindestens einem um Pro handelt, steht, und
Y für Lys oder Arg steht.

2. Insulin-Vorläufer nach Anspruch 1, wobei X₁ für 1-4 Aminosäurereste steht.

3. Insulin-Vorläufer nach Anspruch 1, wobei X₁ für 1-3 oder 1-2 Aminosäurereste steht.

4. Insulin-Vorläufer nach Anspruch 1, wobei X₁ für GluPro und Y für Lys steht.

5. Insulin-Vorläufer nach Anspruch 1, wobei X₁ für AspPro und Y für Lys steht.

6. Insulin-Vorläufer nach Anspruch 1, wobei X₁ für GlnPro und Y für Lys steht.

7. Insulin-Vorläufer nach einem der Ansprüche 1-6, wobei sich ein Pro unmittelbar N-terminal an Y befindet.

8. Polynukleotidsequenz, kodierend einen Insulin-Vorläufer der Formel
B(1-29) - X₁ - Y - A(1-21)
wobei
X₁ für eine Peptidsequenz von 1-5 Aminosäureresten, unter welchen es sich bei mindestens einem um Pro handelt, steht, und
Y für ein Lys oder Arg steht.

9. Verfahren zur Herstellung eines Insulin-Vorläufers, wobei das Verfahren umfasst
(i) Züchten einer Hefezelle, umfassend eine Polynukleotidsequenz, kodierend einen Insulin-Vorläufer der Formel
B(1-29) - X₁ - Y - A(1-21)
wobei
X₁ für eine Peptidsequenz von 1-5 Aminosäureresten, unter welchen es sich bei mindestens einem um Pro handelt, steht, und
Y für Lys oder Arg steht,
unter geeigneten Kulturbedingungen zur Expression des Insulin-Vorläufers;
und
(ii) Isolieren des exprimierten Insulin-Vorläufers.

10. Verfahren zur Herstellung von desB30-Humaninsulin oder Humaninsulin, wobei das Verfahren umfasst
(i) Züchten einer hefezelle, umfassend eine Polynukleotidsequenz, kodierend einen Insulin-Vorläufer der Formel
B(1-29) - X₁ - Y - A(1-21)
wobei
X₁ für eine Peptidsequenz von 1-5 Aminosäureresten, unter welchen es sich bei mindestens einem um Pro handelt, steht, und
Y für Lys oder Arg steht,
unter geeigneten Kulturbedingungen zur Expression des Insulin-Vorläufers;
und
(ii) Isolieren des Insulin-Vorläufers aus dem Kulturmedium und
(iii) Umwandeln des Insulin-Vorläufers in desB30-Humaninsulin oder Humaninsulin durch eine chemische oder enzymatische in-vitro-Umwandlung.

11. Verfahren nach Anspruch 10, wobei X₁ für 1-4 Aminosäurereste steht.

12. Verfahren nach Anspruch 10, wobei X₁ für 1-3 Aminosäurereste steht.

13. Verfahren nach Anspruch 10, wobei X₁ für 1-2 Aminosäurereste steht.

14. Verfahren nach einem der Ansprüche 10-13, wobei sich ein Pro unmittelbar N-terminal an Y befindet.

15. Verfahren nach Anspruch 10, wobei X₁ für GluPro und Y für Lys steht.

16. Verfahren nach Anspruch 10, wobei X₁ für AspPro und Y für Lys steht.

17. Verfahren nach Anspruch 10, wobei X₁ für GlnPro und Y für Lys steht.

## Revendications

1. Précurseur de l'insuline ayant la formule
B(1-29) - X₁ - Y - A(1-21)
dans laquelle
X₁ est une séquence peptidique de 1-5 résidus d'acides aminés, dont au moins l'un est Pro, et
Y est Lys ou Arg.

2. Précurseur de l'insuline selon la revendication 1, dans lequel X₁ est constitué de 1-4 résidus d'acides aminés.

3. Précurseur de l'insuline selon la revendication 1, dans lequel X₁ est constitué de 1-3 ou de 1-2 résidus d'acides aminés.

4. Précurseur de l'insuline selon la revendication 1, dans lequel X₁ est Glu-Pro et Y est Lys.

5. Précurseur de l'insuline selon la revendication 1, dans lequel X₁ est AspPro et Y est Lys.

6. Précurseur de l'insuline selon la revendication 1, dans lequel X₁ est GlnPro et Y est Lys.

7. Précurseur de l'insuline selon l'une des revendications 1-6, dans lequel un Pro est immédiatement N-terminal par rapport à Y.

8. Séquence polynucléotidique codant pour un précurseur de l'insuline ayant la formule
B(1-29) - X₁ - Y - A(1-21)
dans laquelle
X₁ est une séquence peptidique de 1-5 résidus d'acides aminés, dont au moins l'un est Pro, et
Y est Lys ou Arg.

9. Procédé de préparation d'un précurseur de l'insuline, ledit procédé comprenant :
(i) la culture d'une cellule de levure comprenant une séquence polynucléotidique codant pour un précurseur de l'insuline ayant la formule
B(1-29) - X₁ - Y - A(1-21)
dans laquelle
X₁ est une séquence peptidique de 1-5 résidus d'acides aminés, dont au moins l'un est Pro, et
Y est Lys ou Arg,
dans les conditions de culture appropriées à l'expression dudit précurseur de l'insuline ; et
(ii) l'isolement du précurseur de l'insuline exprimé.

10. Procédé de préparation de l'insuline humaine desB30 ou de l'insuline humaine, ledit procédé comprenant
(i) la culture d'une cellule de levure comprenant une séquence polynucléotidique codant pour un précurseur de l'insuline, ayant la formule
B(1-29) - X₁ - Y - A(1-21)
dans laquelle
X₁ est une séquence peptidique de 1-5 résidus d'acides aminés, dont au moins l'un est Pro, et
Y est Lys ou Arg,
dans des conditions de culture appropriées à l'expression dudit précurseur de l'insuline,
(ii) l'isolement du précurseur de l'insuline à partir du milieu de culture, et
(iii) la conversion du précurseur de l'insuline en insuline humaine desB30 ou en insuline humaine par conversion chimique ou enzymatique in vitro.

11. Procédé selon la revendication 10, dans lequel X₁ est constitué de 1-4 résidus d'acides aminés.

12. Procédé selon la revendication 10, dans lequel X₁ est constitué de 1-3 résidus d'acides aminés.

13. Procédé selon la revendication 10, dans lequel X₁ est constitué de 1-2 acides aminés.

14. Procédé selon l'une des revendications 10-13, dans lequel un Pro est immédiatement N-terminal par rapport à Y.

15. Procédé selon la revendication 10, dans lequel X₁ est GluPro et Y est Lys.

16. Procédé selon la revendication 10, dans lequel X₁ est AspPro et Y est Lys.

17. Procédé selon la revendication 10, dans lequel X₁ est GlnPro et Y est Lys.
